# EUROPEAN PATENT SPECIFICATION

(11) **EP 4 013 469 B1**
(45) Date of publication and mention of the grant of the patent: **22.04.2026**
(21) Application number: 20760787.0
(22) Date of filing: 17.08.2020
(51) Int. Cl.: A61L 27/42, A61L 27/44

(54) **MESENCHYMAL STROMAL CELL BONE GRAFT MATERIAL**
MESENCHYMALES STROMAZELLEN-KNOCHENTRANSPLANTATMATERIAL
MATÉRIAU DE GREFFE OSSEUSE À CELLULES STROMALES MÉSENCHYMATEUSES

(30) Priority: 16.08.2019 EP 19192111
(43) Date of publication of application: 22.06.2022
(73) Proprietor: Johann Wolfgang Goethe-Universität Frankfurt am Main, 60323 Frankfurt am Main (DE)
(72) Inventor: HENRICH, Dirk, 60486 Frankfurt am Main (DE); MARZI, Ingo, 61462 Königstein (DE); BADER, Peter, 63303 Dreieich (DE); KUÇI, Selim, 60599 Frankfurt am Main (DE); KUÇI, Zyrafete, 60599 Frankfurt am Main (DE); KLINGEBIEL, Thomas, 60385 Frankfurt am Main (DE); BÖNIG, Halvard, 60437 Frankfurt (DE); SEIFRIED, Erhard, 60320 Frankfurt am Main (DE)
(74) Representative: Kuttenkeuler, David
(86) International application number: PCT/EP2020/073012
(87) International publication number: WO 2021/032688

(56) References cited:
- WO-A1-2016/008895
- WO-A1-2017/173280
- WO-A2-2009/125402
- US-A1- 2008 033 572
- XU XIA ET AL: "Effects of cryopreservation on human mesenchymal stem cells attached to different substrates : Effects of cryopreservation on hMSCs attached to different substrates", JOURNAL OF TISSUE ENGINEERING AND REGENERATIVE MEDICINE, vol. 8, no. 8, 25 July 2014 (2014-07-25), US, pages 664 - 672, XP093064621, ISSN: 1932-6254, DOI: 10.1002/term.1570
- KATSEN-GLOBA ALISA ET AL: "Towards ready-to-use 3-D scaffolds for regenerative medicine: adhesion-based cryopreservation of human mesenchymal stem cells attached and spread within alginate-gelatin cryogel scaffolds", JOURNAL OF MATERIALS SCIENCE: MATERIALS IN MEDICINE, vol. 25, no. 3, 3 December 2013 (2013-12-03), New York, pages 857 - 871, XP093064628, ISSN: 0957-4530, Retrieved from the Internet <URL:http://link.springer.com/article/10.1007/s10856-013-5108-x/fulltext.html> DOI: 10.1007/s10856-013-5108-x
- SKOVRLJ BRANKO ET AL: "Cellular bone matrices: viable stem cell-containing bone graft substitutes", THE SPINE JOURNAL, vol. 14, no. 11, 1 November 2014 (2014-11-01), AMSTERDAM, NL, pages 2763 - 2772, XP093064820, ISSN: 1529-9430, DOI: 10.1016/j.spinee.2014.05.024
- MARQUEZ-CURTIS LEAH A ET AL: "Mesenchymal stromal cells derived from various tissues: Biological, clinical and cryopreservation aspects", CRYOBIOLOGY, ACADEMIC PRESS INC, US, vol. 71, no. 2, 14 July 2015 (2015-07-14), pages 181 - 197, XP029278225, ISSN: 0011-2240, DOI: 10.1016/J.CRYOBIOL.2015.07.003
- COSTA PEDRO F. ET AL: "Cryopreservation of Cell/Scaffold Tissue-Engineered Constructs", TISSUE ENGINEERING. PART C, METHODS DEC 2008, vol. 18, no. 11, 1 November 2012 (2012-11-01), US, pages 852 - 858, XP093064875, ISSN: 1937-3384, DOI: 10.1089/ten.tec.2011.0649
- XU XIA ET AL: "Effects of cryopreservation on human mesenchymal stem cells attached to different substrates : Effects of cryopreservation on hMSCs attached to different substrates", JOURNAL OF TISSUE ENGINEERING AND REGENERATIVE MEDICINE, vol. 8, no. 8, 25 July 2014 (2014-07-25), US, pages 664 - 672, XP93064621, ISSN: 1932-6254, DOI: 10.1002/term.1570
- KATSEN-GLOBA ALISA ET AL: "Towards ready-to-use 3-D scaffolds for regenerative medicine: adhesion-based cryopreservation of human mesenchymal stem cells attached and spread within alginate-gelatin cryogel scaffolds", JOURNAL OF MATERIALS SCIENCE: MATERIALS IN MEDICINE, vol. 25, no. 3, 3 December 2013 (2013-12-03), New York, pages 857 - 871, XP93064628, ISSN: 0957-4530, Retrieved from the Internet <URL:http://link.springer.com/article/10.1007/s10856-013-5108-x/fulltext.html> DOI: 10.1007/s10856-013-5108-x
- SKOVRLJ BRANKO ET AL: "Cellular bone matrices: viable stem cell-containing bone graft substitutes", THE SPINE JOURNAL, vol. 14, no. 11, 1 November 2014 (2014-11-01), AMSTERDAM, NL, pages 2763 - 2772, XP93064820, ISSN: 1529-9430, DOI: 10.1016/j.spinee.2014.05.024
- MARQUEZ-CURTIS LEAH A ET AL: "Mesenchymal stromal cells derived from various tissues: Biological, clinical and cryopreservation aspects", CRYOBIOLOGY, ACADEMIC PRESS INC, US, vol. 71, no. 2, 14 July 2015 (2015-07-14), pages 181 - 197, XP029278225, ISSN: 0011-2240, DOI: 10.1016/J.CRYOBIOL.2015.07.003
- COSTA PEDRO F. ET AL: "Cryopreservation of Cell/Scaffold Tissue-Engineered Constructs", TISSUE ENGINEERING. PART C, METHODS DEC 2008, vol. 18, no. 11, 1 November 2012 (2012-11-01), US, pages 852 - 858, XP93064875, ISSN: 1937-3384, DOI: 10.1089/ten.tec.2011.0649
- KARAM ELDESOQI ET AL: "Safety Evaluation of a Bioglass-Polylactic Acid Composite Scaffold Seeded with Progenitor Cells in a Rat Skull Critical-Size Bone Defect", PLOS ONE, vol. 9, no. 2, 3 February 2014 (2014-02-03), pages e87642, XP055650728, DOI: 10.1371/journal.pone.0087642
- DIRK HENRICH ET AL: "Characterization of Bone Marrow Mononuclear Cells on Biomaterials for Bone Tissue Engineering In Vitro", BIOMED RESEARCH INTERNATIONAL, vol. 2015, 1 January 2015 (2015-01-01), pages 1 - 12, XP055650729, ISSN: 2314-6133, DOI: 10.1155/2015/762407
- KARAM ELDESOQI ET AL: "High Calcium Bioglass Enhances Differentiation and Survival of Endothelial Progenitor Cells, Inducing Early Vascularization in Critical Size Bone Defects", PLOS ONE, vol. 8, no. 11, 14 November 2013 (2013-11-14), pages e79058, XP055651136, DOI: 10.1371/journal.pone.0079058
- CHRISTOPH NAU ET AL: "Treatment of Large Bone Defects with a Vascularized Periosteal Flap in Combination with Biodegradable Scaffold Seeded with Bone Marrow-Derived Mononuclear Cells: An Experimental Study in Rats", TISSUE ENGINEERING PART A, vol. 22, no. 1-2, 1 January 2016 (2016-01-01), US, pages 133 - 141, XP055651138, ISSN: 1937-3341, DOI: 10.1089/ten.tea.2015.0030
- SEEBACH C ET AL: "Comparison of six bone-graft substitutes regarding to cell seeding efficiency, metabolism and growth behaviour of human mesenchymal stem cells (MSC) in vitro", INJURY, JOHN WRIGHT AND SONS, BRISTOL, GB, vol. 41, no. 7, 1 July 2010 (2010-07-01), pages 731 - 738, XP027121266, ISSN: 0020-1383, [retrieved on 20100315]
- CAROLINE SEEBACH ET AL: "Endothelial Progenitor Cells and Mesenchymal Stem Cells Seeded onto [beta]-TCP Granules Enhance Early Vascularization and Bone Healing in a Critical-Sized Bone Defect in Rats", TISSUE ENGINEERING PART A, vol. 16, no. 6, 1 June 2010 (2010-06-01), US, pages 1961 - 1970, XP055651122, ISSN: 1937-3341, DOI: 10.1089/ten.tea.2009.0715

## Description

### FIELD OF THE INVENTION

The invention pertains to the use of mesenchymal stromal cells (MSC) in the treatment of bone disorders or injuries. MSC and preparations of specifically pooled MSC may be used in the manufacturing of bone graft material for implanting into or attaching to bones in order to enhance bone regeneration after surgery or injury, or to treat various bone disorders, such as osteonecrosis. The invention provides a method for producing cryopreserved bone graft material.

### DESCRIPTION

Bone formation and degradation are tightly regulated by growth factor signaling between osteoblasts that are responsible for bone formation and osteoclasts that are responsible for bone re-absorption. Coupling bone formation by osteoblasts with degradation by osteoclasts has recently become a topic of intense study; with the list of growth factors identified as coupling factors expanding. Coupling bone formation with bone re-absorption requires the recruitment of osteoblasts and osteoclasts in parallel with the recruitment of their respective progenitor cells. Osteoblasts derive from mesenchymal stem cell (MSC) while osteoclasts derive from monocytes that are a part of the myeloid-lineage; however, it remains unknown how MSC or monocytes migrate from their niche in the bone marrow to sites of new bone formation. The current understanding of the spatial and temporal regulation of osteogenesis proposes that MSC migrate from their bone marrow niche to the endosteal surface; where the MSC differentiate into osteoblasts that produce new bone. In parallel, monocytes also migrate from their bone marrow niche to the endosteal surface; where they subsequently differentiate into osteoclasts that re-absorb bone. Growth factors known to regulate bone formation include TGFβ-, BMP- and the canonical Wnt-ligands. Osteoclast formation from monocyte precursors and bone re-absorption are regulated through the expression of MSCF, OPG and RANK-ligand. In parallel, osteoclast activity is also regulated by the expression of the TGFβ-, BMP- and the non-canonical Wnt-ligands. However, many developmental growth factors involved in tissue patterning, including TGFβ-, BMP- and the Wnt-ligands, promote bone formation and re-absorption. The maintenance of healthy bone requires constant remodeling, in which bone is made and destroyed continuously.

The introduction of an implant into bone results in a biochemical cascade that drives the pro-inflammatory response that is partially mediated by macrophage activity, which are derived from the myeloid lineage and can contribute to the degradation of bone or an implant material. Currently implants and implant materials are chosen to minimize the macrophage response while being optimally osteo-conductive and promoting maximum bone-implant integration. Alternatively, the introduction of autograft with an implant or the use of devitalized bone tissue graft (autograft) has been employed in concert with the material properties of an implant as a means of increasing osteo-integration; however, these approaches have often been problematic. Ideally, materials could be designed to be both self-organizing and self-assembling.

MSCs are stromal cells that are plastic-adherent and able to differentiate into osteoblasts, chondroblasts, and adipocytes. They express biomarkers including CD73, CD90, and CD105, and they must not express CD14 or CD11b, CD34. MSC can be derived mainly from bone marrow, but also umbilical cord blood or from other sources. In particular bone marrow derived MSC have been most extensively studied both in preclinical and clinical experiments: the effects of such MSC in fracture healing, spinal fusion, and osteonecrosis have been demonstrated (Su P, et al, Int J Mol Sci. 2018 Aug 9;19(8)).

The clinical potential of MSC in the treatment of bone diseases such as osteonecrosis was shown in Mueller et al (Leukemia (2008) 22, 2054-2061). A three week in vitro expansion of autologous MSC was used in patients suffering from steroid induced osteonecrosis. The treatment was safe and resulted in clinical improvement of patients.

Goomez-Barrena et al, (Biomaterials 196 (2019) 100-108) show a successful European, multicentric, first in human clinical trial using calcium phosphate bioceramic granules associated during surgery with autologous mesenchymal stromal cells expanded from bone marrow (BM-hMSC) under good manufacturing practices, in patients with long bone pseudarthrosis. In this study, in vitro expanded MSC are combined with the bone graft material during surgery and implanted into the bone.

Allogenic bone grafts containing live mesenchymal stem cells (MSCs), also known as cellular bone matrices (CBMs) have also been described in the literature. An evaluation of these products is reviewed in Skovrlj, Branko et al, (Spine J. 2014 November 1; 14(11): 2763-2772).

However, still a limiting factor for the use of MSC in the treatment of bone disorders and injury is the long lasting in vitro preparation of autologous MSC. Autologous bone marrow samples are obtained from a patient, MSCs are in vitro isolated therefrom and expanded to obtain sufficient cells for engraftment. The procedure takes weeks in order to obtain MSC preparations usable for implantation. Hence, it was an object of the present invention to develop an "off-the shelf" MSC functionalized bone graft material.

### BRIEF DESCRIPTION OF THE INVENTION

The present invention provides:

A method for producing cryopreserved bone graft material, comprising the steps of
(i) Seeding biological cell material on a scaffold material to obtain a bone graft material, and
(ii) Cryopreserving the bone graft material obtained in the first step,
   wherein the time span between seeding and cryopreservation varies in a cell type dependent manner from 0 min to 24 h, and wherein the scaffold material comprises β-TCP powder, particles or granules.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The invention provides a method for producing cryopreserved bone graft material, comprising the steps of
(i) Seeding biological cell material on a scaffold material to obtain a bone graft material, and
(ii) Cryopreserving the bone graft material obtained in (i),
   wherein the time span between seeding and cryopreservation varies in a cell type dependent manner from 0 min to 24 h, and wherein the scaffold material comprises β-TCP powder, particles or granules.

Bone graft material obtained according to the method of the invention may be used in the treatment of a subject.

The term "cell material" as used in context of the present invention shall in particular pertain to materials comprising biological cell material, such as living or dead cells, preferably living cells, as well as any secreted factors derived from the culturing of such cells. In certain preferred embodiments, however, the present invention pertains to cell material comprising living biological cells. The expression "biological cell" in context of the invention shall preferably refer to a mammalian cell, most preferably a human cell. Certain embodiments of the invention pertain to mesenchymal stromal cells as preferred biological cells in context of the invention. This may in some embodiments exclude other cells that can be derived from bone marrow fractions, such as for example hematopoietic progenitor cells, or erythropoietic progenitor cells. In the present disclosure, the term "mesenchymal stromal cell" is a multipotent stromal cell that can differentiate into a variety of cell types, including, but not limited to osteoblasts (bone cells), chondrocytes (cartilage cells), myocytes (muscle cells) and adipocytes (fat cells). These cells are also known as "mesenchymal stem cells" due to their multi-potency. This biologically important cell population is able to support hematopoiesis, can differentiate along mesenchymal and non-mesenchymal lineages in vitro, is capable of suppressing alloresponses and appears to be non-immunogenic. MSC are, in particular, in the context of the present invention, useful due to their regenerative function by paracrine or endocrine mechanisms, their immune-suppressive activity as well as their ability to form direct cell-to-cell interactions. These cells are known to either secrete certain proteins and/or compounds, which is turn influence the microenvironment around the cells, or when not directly expressing the proteins themselves, mesenchymal stromal cells are known to influence the downstream expression of other proteins, which for example, may be affected by the presence or absence of a mesenchymal stromal cell. That is to say that the presence of a mesenchymal stromal cell within the environment of another cell can cause the other cell to start producing, or even secreting, certain proteins. Therefore, disclosed herein are both compounds and/or proteins secreted by the mesenchymal stromal cells themselves and are therefore considered to be derived from MSCs as they are specifically obtained from MSCs, as well as proteins and compounds that are produced and/or secreted as a result of the presence of mesenchymal stromal cells within the cell environment. Included therein is also the downstream expression of proteins that is initiated via one or more expression cascades due to the presence of mesenchymal stromal cells in the microenvironment. MSC according to the invention may be obtained from a variety of sources such as bone marrow, adipose tissue, umbilical cord blood and others.

The MSC according to the invention is in some preferred embodiments provided as a monogenic composition, therefore, such composition comprises preferably MSC of only one genetic background (or derived from one single donor subject). Other embodiments, which might be preferred in context of the invention, then pertain to a MSC preparation comprising MSC derived from multiple genetically distinct donor subjects. Preferred MSC preparations are those which are prepared according to the methods described in PCT/EP2015/066083 (published as WO 2016/008895; and specifically the method for generating MSC preparations comprising a step of pooling MSC from more than one genetically distinct donor before MSC isolation from such cell pool). The number of donors providing the samples to be pooled for later MSC isolation is preferably more than one and preferably from at least 3, 4, 5, 6, 7, 8, 9 or 10 or more genetically distinct donor subjects. Preferably, MSC according to the present invention are obtained from bone marrow samples, such as bone marrow mononuclear cell (BMNC) fractions.

Hence, in certain preferred embodiments of the invention the cell material is allogenic to the subject to be treated with the bone graft material obtained according to the method of the invention. As used herein, the term "allogenic" or "allogeneic" refers to cells, tissues or organs that are not genetically identical or are derived from a non-genetically identical source to the transplant recipient (e.g., a non-related donor), especially with respect to antigens or immunological reactions. Such cells, tissues or organs are called allografts, allogeneic transplants, homografts or allotransplants.

In certain preferred embodiments the invention, the scaffold material is suitable for implantation into or attachment onto a mammalian bone. The term "scaffold material" is intended to refer to a composition that is capable of forming a scaffold, i.e. a pre-scaffold material. For example, the scaffold material may comprise a composition that is capable of setting into a scaffold. The scaffold material itself may or may not have a structure of a scaffold until the scaffold material has formed the scaffold according to the methods herein. Reference to "a composition that is capable of forming a scaffold" may include the capability to form a scaffold with no further intervention/process steps or components. In an alternative embodiment, reference to "a composition that is capable of forming a scaffold" may include the capability to form a scaffold following further intervention/process steps according to the invention herein and/or following addition of components according to the invention herein. The term "scaffold" (may be interchanged with the term "matrix") is understood to mean a solid mass of material having a 3 - dimensional structure, which may for example be suitable to support cells. In embodiments of the invention, the scaffold may be porous, having interconnected pores or gaps.

According to the invention, the scaffold material comprises β-tricalciumphosphate (TCP) powder, particle or granules. In certain embodiments of the invention, the scaffold material is synthetic or natural, preferably wherein the scaffold material further comprises calcium phosphate, preferably α-TCP, or comprises polylactic acid or polycarpolactone (or other biodegradable polymer), or a mixture of these materials. Natural scaffold material is for example obtained from bones or dental material. Naturally-derived bone scaffold materials are usually prepared by acid extraction of most of the mineralized component to result in so called demineralized bone matrix (DBM). Examples for naturally-derived materials are Bio-Oss^{®} of the mineral portion of bovine bone or Algipore^{®} a porous calcium phosphate material of algae. Autologous (or allogenic) bone is an ideal source of graft material, not only due to its biocompatibility, but also because natural bone grafts facilitate reossification of the defect site by promoting or conducting ingrowth of the patient's own bone tissue to the defect site. Methods for obtaining such material are well known to the person of skill in the art. An additional preferred source of natural bone scaffold material is the use of heat-inactivated spongiosa which is prepared by removing cartilage from a femoral head and heating it to more than 60°C, preferably to more than 80°C, preferably about 90°C, for more than 10 minutes (preferably about an hour).

The aforementioned β-TCP is one crystal form of a composition represented by Ca₃(PO₄)₂, which is a biocompatible compound that is stable at normal temperature. As the β-TCP, commercially available β-TCP powders and β-TCP blocks, for example, β-TCP blocks to be used as scaffold material may be used, or the β-TCP can be produced by a publicly known method (Japanese unexamined Patent Application Publication No. 2004-26648, etc.) or from an α-TCP powder (Japanese unexamined Patent Application Publication No. 2006-89311, etc.). Also, while the β-TCP composition of the present invention may be a β-TCP composition substantially composed of β-TCP or a β-TCP composition produced by mixing in a surfactant, water and so on, the β-TCP composition may be a β-TCP powder obtained by processing a β-TCP block into particles or granules, and besides that, the β-TCP composition is preferably a polycrystalline, high-strength and high-density β-TCP dense body having a low porous rate obtained by densificating a β-TCP powder by tablet pressing or after defoaming by sintering, and among such β-TCP dense bodies, one having a porous rate of 50% or more is preferable. In the present invention, a β-TCP dense body refers to β-TCP having a porous rate of 50% or more. Also in the present invention, powders, particles, and granules all indicate the granular form, and there is no substantial distinction among them. Pores in the β-TCP dense body are determined according to the size and number of spaces between the particles of the polycrystal.

The porous rate of β-TCP can be measured by using a micro CT (Bertoldi S, Farè S, Tanzi MC. Assessment of scaffold porosity: the new route of micro-CT. J Appl Biomater Biomech. 2011 Sep-Dec;9(3):165-75. doi: 10.5301/JABB.2011.8863. Review) a mercury intrusion method, in which pressure is applied to force mercury into a pore and the specific surface area and pore distribution are obtained from the pressure applied and the amount of mercury intruded, or from the density using a calibration curve. It is also possible to measure the true density ρ of a β-TCP stone having a porous rate of 0% (a β-TCP sintered body having the same composition as the sintered body of an object to be measured but having no pores in the crystalline form) in advance, and from an apparent density ρ' calculated from the volume and weight of the sintered body of an object to be measured, calculate the porous rate P(%) as P(%)=(1-ρ' /ρ)×100. Further, the porous rate P can also be measured using, for example, the ACCUPYC 1330 series (the product of Shimadzu Corporation). The porous rate in the β-TCP block as measured by the mercury intrusion method is preferably 50 to 90%, more preferably 60 to 85%, and even more preferably 70 to 80%. A β-TCP powder having a particle diameter of 75 to 105 µm can be produced by pulverizing a β-TCP block, and the porous rate of each powder thus produced as measured by the mercury intrusion method is preferably 50 to 90%, more preferably 55 to 85%, and even more preferably 57 to 80%, and a preferable average porous rate is 60 to 70%. Also, a β-TCP microgranule having a particle diameter of 25 µm or less can also be produced by pulverizing a β-TCP block into particles. The porous rate of the β-TCP microgranule thus produced as measured by the mercury intrusion method is preferably 50% or more, more preferably 50% to 90%. Examples of the β-TCP dense body can include a β-TCP powder, particle, granule, tablet, and columnar body having a porous rate of 50% or more. These β-TCP dense bodies may consist exclusively of β-TCP or comprise β-TCP as scaffold for the MSC seeded on the material as active ingredient, as well as other components.

For example, densification of a β-TCP powder by sintering is performed by sintering preferably at above 600 °C to 2000 °C, more preferably at 750 to 1500 °C, and even more preferably at 900 to 1300 °C, and preferably for 1 to 100 hours, more preferably for 10 to 80 hours, and even more preferably for 20 to 50 hours. Further, sintering can be divided into multiple operations and performed at a plurality of different temperatures. For example, a rod-shaped β-TCP dense body can also be produced by the following method. A slurry is prepared by mixing calcium hydrogen phosphate, calcium carbonate, and water at an appropriate ratio, and the slurry thus prepared is allowed to undergo reaction while grinding, followed by drying. Subsequently, the solid material obtained after drying is pulverized and calcined to give a β-TCP powder. The β-TCP powder thus obtained is formed into a columnar body by compression forming. The resulting compression-formed columnar body is sintered at 600 °C to 1300 °C for about 20 hours.

In some embodiments, which might be preferred, the scaffold material is a nanoporous material. As used herein, the term "nanoporous" generally refers to a porous material (i.e. a calcium phosphate ceramic) whose average pore diameter is in the nanometer range (typically between 1 to 1000 nm). More preferably, the pores of the nanoporous material of the invention are communicating nanopores, which preferably are pores which are interconnected via channels. Such communicating pores preferably have a degree of porosity and interconnection that there is a channel connection from one side of the graft to the other. A porosity according to the invention is preferably a porosity of 50% - 90%, preferably of 60% - 70%; and/or wherein the nanopores have an average diameter of 50 - 600 µm, preferably of 100 - 500 µm; and/or wherein the nanopores are communicating nanopores.

In some further embodiments, the scaffold material is preferably not a decellularized tissue matrix obtained from natural, such a xenogeneic (non-human) tissues. The term "decellularized" or "decellularization" as used herein refers to a biostructure (e.g., a tissue or an organ, or part of an organ), from which the cellular content has been removed leaving behind an intact acellular infra-structure mainly composed of collagens.

In certain preferred embodiments of the invention the bone graft material is in the form of an implantable tablet, preferably a tablet having cylindrical shape (rod-shaped). The term "cylindrical shape" as used herein refers to any geometrical shape having the same cross section area throughout the length of the geometrical shape. The cross section of a cylinder within the meaning of the present invention may have any two-dimensional shape, for example the cross section may be circular, oval, rectangular, triangular, angular or star shaped. Pharmaceutical compositions described herein may have a cylindrical shape, wherein the end(s) are optionally tapered. In particular embodiments, a bone graft material obtained according to the method of the invention is a tablet having a height of 1 to 10 mm, preferably 3 to 5 mm, and/or a diameter of 1 to 15 mm, preferably 5 to 8 mm. In context of the herein disclosed treatment of bone disorders and injuries, a tablet with the herein disclosed dimensions showed surprisingly improved effects for the performance of the MSC during the treatment. As used herein, the "length" refers to the dimension of the longest axis of the tablet and the "height" refers to the dimension of the longest axis in the largest plane of the tablet. The diameter therefore refers to the axis perpendicular to the height.

Bone graft material obtained according to the method of the invention is cryopreserved and thus would optionally require thawing immediately before surgical insertion or attachment to the bone of a patient. The term "cryopreservation", as used herein, refers to the process of cooling and storing biological cells, tissues, or organs at low temperatures to maintain their viability. As a non-limiting example, cryopreservation can be the technology of cooling and storing cells at a temperature below the freezing point (e.g., -20 °C or colder, -80 °C or colder, or the like) that permits high rates of survivability of the cells upon thawing. In context of the invention the biological material seeded onto the scaffold material is cryopreserved in order to provide an off-the-shelf product for implantation.

Bone graft material obtained according to the claimed method may be compressed into a bone graft implant in the form of an implantable tablet having a height of 1 to 10 mm, preferably 3 to 5 mm, and/or a diameter of 1 to 15 mm, preferably 5 to 8 mm.

Bone graft implants may be made from allograft, autograft, xenograft, cortical, cancellous, cartilage, and combinations of, synthetic HA, B-TCP, Ceramic, PLA, PLGA, PLLA or other such materials and may be incorporated with growth factors, plasma (e.g., platelet rich plasma), platelets (e.g., intact platelets), cells, peptides or other such materials.

Bone graft implants may be cylinders, rectangular, trapezoidal, flat, convex, concave, and may have a channel on one or both sides for easy placement against a patient's bone or for buttressing against a bone. Bone graft implants may be trimmed to size and snapped onto a rod by press fit or by means of sutures.

Preferably, the bone graft implant biological cell material comprises living cells, preferably allogenic or autologous.

The bone graft material, cellular composition or other compounds, materials and composition described herein preferably do not comprise added, and preferably immobilized, bone growth factors or cytokines. Such growth factors and cytokines are often added in order to render cells in bone graft material osteo-inductive. However, the materials of the present invention in all aspects and embodiments thereof preferably do not require such addition of osteo-inductive cytokines or growth factors since the cellular compositions of the invention are inherently osteo-inductive. Such cytokines include bone morphogenic protein (BMP), Indian hedgehog (IHH), transforming growth factor β (TGF3); bone morphogenetic proteins (BMPs); fibroblast growth factors (FGFs); Wnt ligands and β-catenin; insulin-growth factors (IGFs); collagen-1; Runx2; osteopontin; osterix; vascular endothelial growth factor (VEGF); platelet derived growth factor (PDGF); osteoprotegerin (OPG); NEL-like protein 1 (NELL-1 ); or a mixture thereof.

.

Since freezing a biological sample containing living material such as cells bears the risk of sheering and destruction of the cell material, cryopreservation medium is used in order to minimize the loss of cell material during the process. As used herein, the term "cryopreservation medium" or "cryoprotectant" is a substance that is used to protect eukaryotic cells (and also tissues, organs) from freezing damage. Further, the cryopreservation agent or cryoprotectant may protect the eukaryotic cells (and also tissues or organs) from cold and heat shock, dehydration, and cryo-toxicity during cryopreservation. The cryopreservation agent or cryoprotectant may be cell penetrating or non-penetrating. Non-limiting examples of cryoprotectants include glycerol, DMSO (dimethyl sulfoxide), propylene glycol, ethylene glycol, acetamide, and methanol. However it should be cautioned that cryoprotectants have some disadvantages in that they can induce protein denaturation at higher temperature and cause cryoprotectant toxicity in cellular systems (like tissues and organs). The toxicity of the cryoprotectants is a major limitation to successful cryopreservation of eukaryotic cells. DSMO is a preferred cryoprotectant for use in accordance with the present invention.

Preferably, in some embodiments of the invention the cryopreservation is performed at -15 °C or less, preferably is -20 °C or less, preferably less than -50 °C (about -70 °C or -80 °C); preferably the cryopreservation comprises a step of freezing at a freezing temperature of less than -20 °C, such as -40 °C or less, and subsequent storing of at temperatures higher than the freezing temperature.

In accordance with the present invention the scaffold material for use in the process of the invention is in granulate form, preferably wherein the granules have an average size of 0.1 to 3 mm, preferably 0.5 to 3 mm, more preferably 0.7 to 2.8 mm, more preferably 1.4 to 2.8 mm. However, in other embodiments the scaffold material is already provided in the form of an implantable body, such as a tablet or rod, preferably in cylindrical shape.

The biological cell material is seeded on the scaffold material in accordance with some embodiments of the invention in a density of at least 0.1 x 10⁶ cells per mL scaffold material, preferably of at least 0.5 x 10⁶ cells per mL scaffold material, more preferably of about 1 x 10⁶ cells per mL scaffold material.

A therapeutic kit comprising separate containers of the biological cell material and the scaffold material as described herein, may be provided for carrying out the first step of the claimed method.

The first and the second container may have any form suitable for packaging their respective materials. It might be however preferred that the containers are formed such to allow a direct mixing of the content of the first container with the content of the second container to obtain the final product for use in the treatment. Syringes are one example of such containers.

The therapeutic kit may comprise additional components useful for realizing a medical treatment. Buffers, media, or instructions for use are non-limiting examples of such additional components.

Disclosed but not claimed herein is a mesenchymal stromal cell (MSC), or a MSC preparation for use in the treatment of a disease, wherein the treatment comprises the administration of the MSC, or the MSC preparation, to a subject in need of the treatment by adhering the MSC or MSC preparation to a bone graft material to obtain an MSC-bone graft material, and implanting or attaching the MSC-bone graft material into/to one or more bone(s) of the patient.

Disclosed but not claimed herein is a use of a MSC, or a MSC preparation, in the manufacture of a medicament, preferably a bone graft material, for use in the treatment of a bone injury or disorder. Such bone injuries may be selected from aseptic bone necrosis (spontaneous or therapy induced), bone fractures, fractures with secondary dislocation, pseudo- arthrosis, and reduced and delayed bone build-up. Treatable bone disorder may be any condition affecting the development and/or the structure of the skeletal system. Bone disorders can include any disorder that results in an imbalance in the ratio of bone formation to bone resorption such that, if unmodified, a subject will exhibit less bone than desirable, or a subject's bones will be less intact and coherent than desired. Bone disorders may refer to disorders, diseases, conditions or traumas of bone, such as fractures, osteoporosis, osteoarthritis, or cancers. For example, a bone disorder may result from fracture, from surgical intervention or from dental or periodontal disease. These diseases have a variety of causes and symptoms known in the art. Bone disorders can affect people of all ages, from newborns to older adults. However, certain age groups are more likely to have certain types of disorders.

The uses and methods described herein may comprise a step of culturing the MSC, or MSC preparation and in particular the MSC comprised in the graft material, before implantation. Hence, the uses and methods may comprise a step of culturing for at least 6h, preferably at least 12h, more preferably at least 18h, more preferably at least 24h, even more preferably at least 36h and most preferably at least 48h or more. The indicated culture times are commenced immediately after thawing the frozen MSC preparation and before implantation. Maximum culture times are up to 96h, or less, for example 7h - most preferred is a culturing time of 24 to 72h, or for about 48h.

Hence, a method for thawing a bone graft material according and preparing the bone graft material for implantation, may comprise a step of culturing the MSC comprised in the bone graft material for at least 6h, preferably at least 12h, more preferably at least 18h, more preferably at least 24h, even more preferably at least 36h and most preferably at least 48h or more. The indicated culture times are commenced immediately after thawing the frozen MSC preparation and before implantation. Maximum culture times are up to 96h, or less, for example 7h - most preferred is a culturing time of 24 to 72h, or for about 48h.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

In view of the above, it will be appreciated that the present invention also relates to the particular embodiments as set out in the claims.

### BRIEF DESCRIPTION OF THE FIGURES

The figures show:

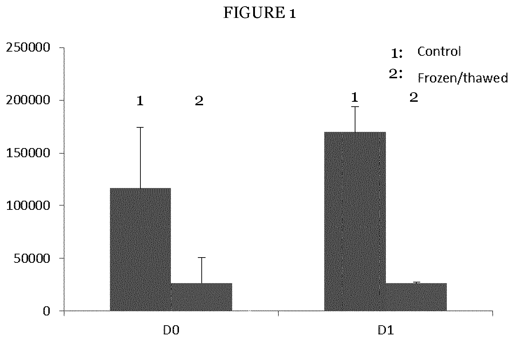

**Figure 1**: shows the metabolic activity of MSC seeded on β-TCP, frozen and thawed (second bars) and of MSC cultured on β-TCP permanently at 37 °C (first bars). Mean values and standard deviation are shown. MSC were from the same donor, measurement was done in triplicate. Metabolic activity was assessed by means of MTT assay.

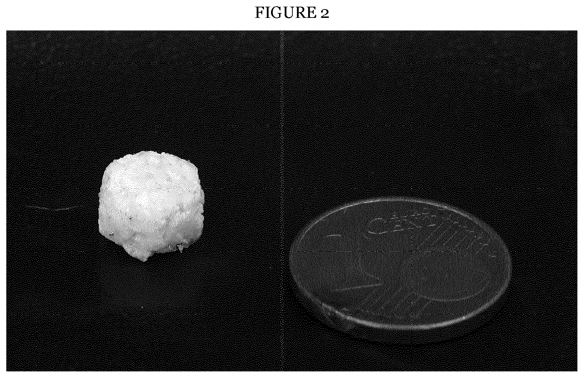

**Figure 2****:** shows a photograph of the tablet of the invention

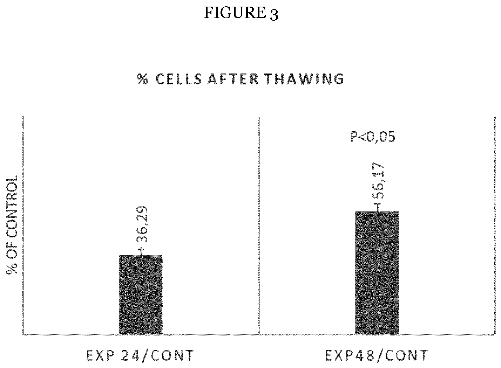

**Figure 3****:** shows human MSC (cell pool of n=8 donors) were placed for 3h on β-TCP granules, which were modelled into the approximate dimensions of the planned MSC tablet (radius 4 mm, height 5 mm) by using a permeable form. MSC were applied at a density of 1x106 cells per cm³ β-TCP according to established seeding protocols and cultivated for 3h at 37°C and then first frozen at -80°C (overnight) and then -196°C (7 days) (experimental group). Metabolic activity was determined by MTT test in parallel cultures 24h and 48h after thawing. The control group consisted of MSC seeded at the same density and in the same manner on β-TCP, but incubated for 24h at 37°C only. Subsequently, the metabolic activity was also determined.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Comparative Example 1: Collection of bone marrow from 8 healthy third-party donors and isolation of BM-MNCs.

After obtaining a written informed consent, from each bone marrow donor were collected up to 250 ml additional bone marrow aspirate for the purpose of MSC banking with approval by the local Ethics Committee in full agreement with the Declaration of Helsinki. In total from 8 donors the inventors obtained 1.66 liters bone marrow. For isolation of bone marrow mononuclear cells by Ficoll-gradient the inventors used the Sepax machine as shown in Figure 1. The absolute number of BM-MNCs per 1 ml of bone marrow after this isolation procedure was 3.3 x 10⁶ ± 6.3 x 10⁵ cells. Total number of BM-MNCs which was obtained from eight donors after two washing steps was 9.86 x 10⁹. These cells were resuspended in cryomedium and distributed in the bags, that were frozen using a rate-controlled freezer and then stored in the vapour phase of liquid nitrogen until use.

### Comparative Example 2: Generation of mesenchymal stromal cells from bone marrow mononuclear cells and establishment of a MSC-bank

To generate the MSC-Bank, bone marrow mononuclear cells from 8 donors were thawed, washed and pooled in DMEM supplemented with 5% PL. To find out the optimal concentration of platelet lysate for the adherence of progenitor cells of MSCs the inventors cultured BM-MNCs with both concentrations of PL: 5% and 10%. The obtained results have demonstrated that the 5% concentration of platelet lysate is much more efficacious in promotion of BM-MNCs and generation of MSCs than 10% concentration of PL. In addition, the inventors asked which of these two concentrations of PLs is better for clinical-scale expansion of MSCs. The inventors found that that the 10% concentration of PLs is significantly more efficient in expanding the MSCs than the 5% PL. Moreover, in both cases the unfiltered platelet lysates were more effective for generation and expansion of MSCs than the filtered ones. These preliminary experiments paved the way for establishing the master MSC-bank. Therefore, the inventors thawed the BM-MNCs from each donor and after washing twice they were pooled together and thereafter cultured for 14 days, as described in the section of methods. The inventors were able to generate from 9.89x10⁹ BM-MNCs 3.2x 10⁸ MSCs of passage 1. These MSCs expressed the typical markers for MSCs, such as CD73, CD90 and CD105 but were negative for hematopoietic cell markers e.g. CD14, CD34, CD45. According to trypan blue staining the viability of these MSCs before freezing was 95 ± 5%.

The total number of MSCs was distributed in 210 cryovials each containing 1.5x10⁶ MSC P1 and finally frozen in the gaseous phase of liquid nitrogen until use. The inventors referred to this set of vials as MSC-bank. The MSC as isolated according to comparative example 2 have improved allo suppressive potential as can be derived from WO 2016/008895.

### Example 1: Scaffold materials

Dimensions and shape: Cylindrical, height 3-5 mm, diameter of 5-8 mm, depending on the size of the bone defect. A number of scaffolds can be combined to fill larger defects as they occur in the treatment of femoral head osteonecrosis.

The scaffold should consist of natural or synthetic matrices e.g. α-TCP, β-TCP, demineralized bone matrix, polylactic acid or polycaprolactone.

The scaffold should preferably be mechanically stable in order to prevent collapsing during surgical procedures and implantation. The scaffold should offer communicating macropores of the size range 100 µm to 500 µm. Pore sizes from 100 µm improve angiogenesis, while pore diameters of 300-400 µm have a positive effect on osteoconductivity. Micropores enabling surface enlargement, cellular adhesion and improvement of nutritional support should range from1 µm to 10 µm. Overall porosity of the scaffold should be 60 - 70%, thus ensuring highest possible degree of porosity without compromising the mechanical strength.

The surface of the material should be rather smooth, rough microstructures in size range 1 - 10 µm should be prevented. Preferably, the surface consists of smooth slightly convex structures with a diameter ranging from 8-15 µm that are arranged in a honey comb similar matter.

### Example 2: Manufacture of a bone implant graft

MSC derived from the cell bank of comparative example 2 were seeded to the scaffolds as described in Henrich et al, 2009, 2013, 2014; Seebach et al 2010, 2012, 2015. In brief, cells were harvested and preferably adjusted to a density of 1*10⁶ cells/mL (Range 1 - 1*10⁷ cells/mL). The cell suspension is carefully dripped on an equal volume of scaffold.

Non adsorbed cell suspension is carefully distributed once again evenly on the scaffold followed by 10 min incubation at 37 °C, 5% CO2, 100% humidity. This procedure (Wetting of scaffold with non-adsorbed cell suspension followed by incubation) will be repeated two more times.

Such obtained functionalized graft material is ready for use or can be cryopreserved.

### Example 3: Cryopreservation of bone graft implants

Cryopreservation will be performed preferably immediately after the seeding procedure but time span between seeding and cryopreservation may vary in a cell type dependent manner from 0 min to 24hrs. In the latter case the scaffold will be stored in medium (DMEM + 5% platelet lysate) at 37 °C, 5% CO2, 100% humidity until cryopreservation procedure takes place.

The cell populated scaffolds were subjected to cryopreservation medium preferably consisting of physiologic NaCl solution (65 % v/v, final NaCl concentration 0.7%), human serum albumin solution (HSA, 25% v/v, final HSA concentration 5%) and DMSO (10% v/v).

The cryopreservation medium is provided in sterile plastic bags. After addition of cell seeded scaffolds (range 1 to 20 scaffolds per bag), the bags are closed by heat sealing. The sealed bags were immediately subjected to a controlled rate freezer. Long term storage will be performed in liquid nitrogen vapor phase. The possibility for short term storage at -30 °C to -20 °C will be analysed.

### Example 4: Thawing and use of bone graft implants

Opening of sterile envelope and immediate subjection of the scaffold to a 50 mL vial filled with room temperature or prewarmed saline + HSA (0,5-5%, cleansing solution) followed by 5 min incubation in order to remove DMSO from the scaffold. The scaffold can be aseptically removed from the cleansing solution and should be placed immediately into the bone defect.

### Example 5: Medical use of bone graft implants

MSC were seeded on approximately 200 µL densely compressed β-TCP granules (size 1.4 -2.8 mm) in a density of 1*10⁶ cells/mL β-TCP- scaffold. The seeding procedure consists of repeated dripping of the cell suspension over the scaffolds during a period of 30 minutes. Subsequently scaffolds were immediately frozen at -80 °C overnight in a medium containing 10% DMSO and 90% FCS. As control, MSC on β-TCP (same donor) were cultivated at 37 °C in parallel. All procedures described in the following were also performed with the control approach. Next day the well plate containing the scaffolds was placed for 3 min in a water bath (37°C) in order to enable recovery of scaffolds. Scaffolds were then immediately dropped into 30 mL prewarmed (room temperature) medium (RPMI+10% FCS) for 1 min and recovered by filtering with cell strainer (100 µm mesh). Scaffolds were equally distributed into a 96-well plate using sterile forceps and 100 µL medium (Mesencult+supplements) were added to each well. In order to assess metabolic activity of the MSC on the scaffold an MTT assay was performed in triplicate following the instructions of the manufacturer. Incubation time with MTT reagent was 4 hrs. In order to assess long term survival of the MSC a portion of cells was cultured for additional 24hrs after thawing followed by an MTT assay.

Metabolic cell equivalent (MCE) of frozen and thawed MSC was 2.7*10⁴ and around 25% of MSC cultured at 37 °C on β-TCP (5.8*104 MCE). If MSC were cultured additional 24hrs after thawing, the metabolic activity remained approximately constant (2.6*10⁴ MCE) compared to cell activity directly measured after thawing (2.7*10⁴ MCE) whereas the metabolic activity of MSC cultured permanently at 37 °C increased further (1.7*10⁵ MCE) compared to the initial value (1.2*10⁵ MCE, Figure 1). These results indicate that MSC seeded on a β-TCP scaffold can be stored frozen at -80 °C and remain in part vital. Furthermore, it can be concluded that frozen/thawed MSC did not further deteriorate 24hrs after thawing, though they did not improve either. One might assume that the freeze/thaw procedure induced a lag phase of MSC proliferation.

### Example 6: Optimization of cell seeding as well as cryopreservation, thawing and reconstitution of the cell-populated scaffolds

Human MSC-Pool (8 donors, passage 2) was obtained from the Clinic for Pediatrics and Adolescent Medicine. The cells were further cultivated up to a maximum of the 5th passage and used in experiments, or cryopreserved for later experiments. MSC phenotype was activated by FACS analysis (CD90+, CD105+, CD34-, CD45-) and the ability for osteogenic differentiation.

Using the MSC pool, our previous results were reproduced in a series of pilot experiments (n=3), which showed that hMSC, seeded on β-TCP scaffold and cultivated for a short time (3 h) at 37°C, are reduced metabolically active after freezing and thawing.

The metabolic activity of the cells was measured on the day after sowing for the control group and 1 and 2 days after rethawing using the MTT test. The mean metabolic activity of the cells of the experimental group increased significantly with increasing cultivation time after thawing (see figure 3: 36.3% of the control on day 1 and 56.2% of the control on day 2 after thawing; p<0.05, n=3).

## Claims

1. **A method for producing cryopreserved bone graft material,** comprising the steps of
**(i)** Seeding biological cell material on a scaffold material to obtain a bone graft material; and
**(ii)** cryopreserving the bone graft material obtained in (i),
wherein the time span between seeding and cryopreservation varies in a cell type dependent manner from 0 min to 24 h, and wherein the scaffold material comprises β-TCP powder, particles or granules.

2. The method of claim 1, wherein the biological cell material comprises mesenchymal stromal cells (MSC) derived from any source, or comprises bone marrow mononuclear cells (BMC).

3. The method of claim 1 or 2, wherein the biological cell material comprises living cells of one donor subject, preferably of more than two genetically distinct donor subjects, preferably from at least 3, 4, 5, 6, 7, 8. 9 or 10 or more genetically distinct donor subjects.

4. The method of any one of claims 1 to 3, wherein the scaffold material is synthetic or natural.

5. The method of any one of claims 1 to 4, wherein the scaffold is in granulate form, preferably wherein the granules have an average size of 0.1 to 3 mm, preferably 0.5 to 3 mm, more preferably 0.7 to 2.8 mm, more preferably 1.4 to 2.8 mm

6. The method of any one of claims 1 to 5, wherein the bone graft material is in the form of a tablet, and the tablet has a height of 1 to 10 mm, preferably 3 to 5 mm, and/or a diameter of 1 to 15 mm, preferably 5 to 8 mm.

7. The method of any one of claims 1 to 6, wherein the cryopreservation is at -15 °C or less, preferably at -20 °C or less, preferably less than -50 °C, more preferably at about -70 °C or -80 °C.

8. The method of any one of claims 1 to 7, wherein the scaffold is porous, preferably with a porosity of 50%-90%.

9. The method according to any one of claims 1 to 8, wherein the biological cell material is seeded on the scaffold material in a density of at least 0.1 x 10⁶ cells per mL scaffold material, preferably of at least 0.5 x 10⁶ cells per mL scaffold material, more preferably of about 1 x 10⁶ cells per mL scaffold material.

## Patentansprüche

1. **Verfahren zum Herstellen eines kryokonservierten Knochentransplantatmaterials,** umfassend die Schritte
(i) Aussäen eines biologischen Zellmaterials auf ein Gerüstmaterial, um ein Knochentransplantatmaterial zu erhalten; und
(ii) Kryokonservieren des Knochentransplantatmaterials, das in (i) erhalten wird,
wobei die Zeitspanne zwischen dem Aussäen und der Kryokonservierung in einer zelltypabhängigen Weise von 0 min bis 24 h variiert und wobei das Gerüstmaterial ein β-TCP-Pulver, -Teilchen oder -Granulate umfasst.

2. Verfahren nach Anspruch 1, wobei das biologische Zellmaterial mesenchymale Stromalzellen (MSC) umfasst, die aus einer beliebigen Quelle abstammen, oder mononukleäre Knochenmarkzellen (BMC) umfasst.

3. Verfahren nach Anspruch 1 oder 2, wobei das biologische Zellmaterial lebende Zellen eines Spendersubjekts umfasst, vorzugsweise von mehr als zwei genetisch eindeutigen Spendersubjekten, vorzugsweise von mindestens 3, 4, 5, 6, 7, 8, 9 oder 10 oder mehr genetisch eindeutigen Spendersubjekten.

4. Verfahren nach einem der Ansprüche 1 bis 3, wobei das Gerüstmaterial synthetisch oder natürlich ist.

5. Verfahren nach einem der Ansprüche 1 bis 4, wobei das Gerüst in einer Granulatform vorliegt, vorzugsweise wobei die Granulate eine durchschnittliche Größe von 0,1 bis 3 mm, vorzugsweise 0,5 bis 3 mm, mehr bevorzugt 0,7 bis 2,8 mm, mehr bevorzugt 1,4 bis 2,8 mm aufweisen

6. Verfahren nach einem der Ansprüche 1 bis 5, wobei das Knochentransplantatmaterial in der Form einer Tablette vorliegt und die Tablette eine Höhe von 1 bis 10 mm, vorzugsweise 3 bis 5 mm, und/oder einen Durchmesser von 1 bis 15 mm, vorzugsweise 5 bis 8 mm aufweist.

7. Verfahren nach einem der Ansprüche 1 bis 6, wobei die Kryokonservierung bei -15 °C oder weniger, vorzugsweise bei -20 °C oder weniger, vorzugsweise weniger als -50 °C, mehr bevorzugt bei etwa -70 °C oder -80 °C erfolgt.

8. Verfahren nach einem der Ansprüche 1 bis 7, wobei das Gerüst porös ist, vorzugsweise mit einer Porosität von 50 %-90 %.

9. Verfahren nach einem der Ansprüche 1 bis 8, wobei das biologische Zellmaterial auf dem Gerüstmaterial in einer Dichte von mindestens 0,1 x 10⁶ Zellen pro mL Gerüstmaterial, vorzugsweise von mindestens 0,5 x 10⁶ Zellen pro mL Gerüstmaterial, mehr bevorzugt von etwa 1 x 10⁶ Zellen pro mL Gerüstmaterial ausgesät wird.

## Revendications

1. **Procédé de production de matériau de greffe osseuse cryoconservée,** comprenant les étapes consistant à
(i) ensemencer du matériau cellulaire biologique sur un matériau d'échafaudage pour obtenir un matériau de greffe osseuse ; et
(ii) cryopréserver le matériau de greffe osseuse obtenu en (i),
dans lequel le laps de temps entre l'ensemencement et la cryoconservation varie de 0 min à 24 h en fonction du type de cellule, et dans lequel le matériau d'échafaudage comprend de la poudre, des particules ou des granulés de β-TCP.

2. Procédé selon la revendication 1, dans lequel le matériau cellulaire biologique comprend des cellules stromales mésenchymateuses (CSM) dérivées d'une quelconque source, ou comprend des cellules mononucléées de moelle osseuse (BMC).

3. Procédé selon la revendication 1 ou 2, dans lequel le matériau cellulaire biologique comprend des cellules vivantes d'un sujet donneur, de préférence de plus de deux sujets donneurs génétiquement distincts, de préférence d'au moins 3, 4, 5, 6, 7, 8, 9 ou 10 sujets donneurs génétiquement distincts ou plus.

4. Procédé selon l'une quelconque des revendications 1 à 3, dans lequel le matériau d'échafaudage est synthétique ou naturel.

5. Procédé selon l'une quelconque des revendications 1 à 4, dans lequel l'échafaudage se présente sous forme de granulés, de préférence dans lequel les granulés ont une taille moyenne de 0,1 à 3 mm, de préférence de 0,5 à 3 mm, plus préférablement de 0,7 à 2,8 mm, plus préférablement de 1,4 à 2,8 mm.

6. Procédé selon l'une quelconque des revendications 1 à 5, dans lequel le matériau de greffe osseuse se présente sous la forme d'un comprimé, et le comprimé a une hauteur de 1 à 10 mm, de préférence de 3 à 5 mm, et/ou un diamètre de 1 à 15 mm, de préférence de 5 à 8 mm.

7. Procédé selon l'une quelconque des revendications 1 à 6, dans lequel la cryoconservation se fait à -15 °C ou moins, de préférence à -20 °C ou moins, de préférence à moins de -50 °C, plus préférablement à environ -70 °C ou -80 °C.

8. Procédé selon l'une quelconque des revendications 1 à 7, dans lequel l'échafaudage est poreux, de préférence avec une porosité de 50 % à 90 %.

9. Procédé selon l'une quelconque des revendications 1 à 8, dans lequel le matériau cellulaire biologique est ensemencé sur le matériau d'échafaudage à une densité d'au moins 0,1 x 10⁶ cellules par mL de matériau d'échafaudage, de préférence d'au moins 0,5 x 10⁶ cellules par mL de matériau d'échafaudage, plus préférablement d'environ 1 x 10⁶ cellules par mL de matériau d'ossature.
